# EUROPEAN PATENT APPLICATION

(11) **EP 4 385 479 A1**
(43) Date of publication of application: **19.06.2024**
(21) Application number: 22870122.3
(22) Date of filing: 20.07.2022
(51) Int. Cl.: A61H 1/02, A61H 37/00, G01S 19/13, G16H 10/60, G16H 40/60, G16H 20/30

(54) **ELECTRONIC DEVICE AND METHOD FOR CONTROLLING AT LEAST ONE WEARABLE DEVICE, AND NON-TRANSITORY COMPUTER READABLE STORAGE MEDIUM**

(30) Priority: 18.09.2021 KR 20210125319; 21.10.2021 KR 20210141418
(71) Applicant: Samsung Electronics Co., Ltd., Suwon-si, Gyeonggi-do 16677 (KR)
(72) Inventor: LEE, Seungjoon, Suwon-si Gyeonggi-do 16677 (KR); LEE, Sangyoon, Suwon-si Gyeonggi-do 16677 (KR)
(74) Representative: HGF
(86) International application number: PCT/KR2022/010626
(87) International publication number: WO 2023/043035

(57) **Abstract**

An electronic device according to an embodiment may obtain first movement information about the electronic device and second movement information about an external electronic device. The electronic device may identify, on the basis of the first movement information about the electronic device and the second movement information about the external electronic device, a first mode that is a mode in which a first wearable device operates using a first actuator, and a second mode that is a mode in which a second wearable device operates using a second actuator. The electronic device may transmit a request signal to the first wearable device to operate in the first mode using the first actuator. The electronic device may transmit a request signal to the external electronic device to operate in the second mode.

## Description

### [Technical Field]

The following descriptions relate to an electronic device, method, and non-transitory computer readable storage medium for controlling at least one wearable device.

### [Background Art]

A walking assistance device is a device that assists a user who has difficulty walking and allows the user to walk easily. The walking assistance device was developed to relieve the inconvenience of walking.

Recently, interest in a walking assistance device for exercise has been increasing. The walking assistance device may generate a load on a movement of a user or reduce a load on a movement of the user. The walking assistance device may be controlled by an electronic device of the user, and may operate at an intensity desired by the user.

### [Disclosure]

### [Technical Problem]

An electronic device may establish a connection with a wearable device (e.g., the walking assistance device). The electronic device may control only the connected wearable device and may not control a wearable device connected to an external electronic device. Therefore, an organic operation of the wearable device connected to the electronic device and the wearable device connected to the external electronic device may not be performed. Therefore, a method for performing the organic operation of the wearable device connected to the electronic device and the wearable device connected to the external electronic device may be required.

Technical tasks to be achieved in the present disclosure are not limited to the technical tasks mentioned above, and other technical tasks not mentioned will be clearly understood by those having ordinary knowledge in the art to which the present disclosure belongs from the description below.

### [Technical Solution]

According to an embodiment, an electronic device may comprise at least one memory configured to store instructions, global positioning system (GPS) circuit, at least one communication circuit, and at least one processor operably coupled to the at least one memory, the GPS circuit, and the at least one communication circuit. The at least one processor may be configured, when executing the instructions, to establish a first connection with a first wearable device which comprises a first actuator and is worn by a first user of the electronic device. The at least one processor may be configured to establish a second connection with an external electronic device connected with a second wearable device which comprises a second actuator and is worn by a second user. The at least one processor may be configured, while the first connection and the second connection are maintained, to obtain first movement information of the electronic device and second movement information of the external electronic device. Based on the first movement information and the second movement information, the at least one processor may be configured to identify a first mode in which the first wearable device operates using the first actuator and a second mode in which the second wearable device operates using the second actuator. The at least one processor may be configured to transmit, through the first connection, a request signal for operating the first mode to the first wearable device. The at least one processor may be configured to transmit, through the second connection, a request signal for operating the second mode to the external electronic device.

According to an embodiment, a method of an electronic device may comprise establishing a first connection with a first wearable device which comprises a first actuator and is worn by a first user of the electronic device. The method may comprise establishing a second connection with an external electronic device connected with a second wearable device which comprises a second actuator and is worn by a second user. The method may comprise, while the first connection and the second connection are maintained, obtaining first movement information of the electronic device and second movement information of the external electronic device. The method may comprise, based on the first movement information and the second movement information, identifying a first mode in which the first wearable device operates using the first actuator and a second mode in which the second wearable device operates using the second actuator. The method may comprise transmitting, through the first connection, a request signal for operating the first mode to the first wearable device. The method may comprise transmitting, through the second connection, a request signal for operating the second mode to the external electronic device.

According to an embodiment, a non-transitory computer readable storage medium may store one or more programs comprising instructions, which, when being executed by at least one processor of an electronic device with global positioning system (GPS) circuit and at least one communication circuit, cause the electronic device to establish a first connection with a first wearable device which comprises a first actuator and is worn by a first user of the electronic device. The non-transitory computer readable storage medium may store one or more programs comprising instructions which cause the electronic device to establish a second connection with an external electronic device connected with a second wearable device which comprises a second actuator and is worn by a second user. The non-transitory computer readable storage medium may store one or more programs comprising instructions which cause the electronic device to obtain first movement information of the electronic device and second movement information of the external electronic device, while the first connection and the second connection are maintained. Based on the first movement information and the second movement information, the non-transitory computer readable storage medium may store one or more programs comprising instructions which cause the electronic device to identify a first mode in which the first wearable device operates using the first actuator and a second mode in which the second wearable device operates using the second actuator. The non-transitory computer readable storage medium may store one or more programs comprising instructions which cause the electronic device to transmit, through the first connection, a request signal for operating the first mode to the first wearable device. The non-transitory computer readable storage medium may store one or more programs comprising instructions which cause the electronic device to transmit, through the second connection, a request signal for operating the second mode to the external electronic device.

### [Advantageous Effects]

An electronic device according to an embodiment may obtain first movement information of the electronic device and second movement information of an external electronic device. The electronic device may identify, based on the first movement information of the electronic device and the second movement information of the external electronic device, a first mode in which a first wearable device connected with the electronic device will operate and a second mode in which a second wearable device will operate. The electronic device may transmit a request signal for operating the first mode to the first wearable device and a request signal for operating the second mode to the external electronic device. The electronic device may control not only the first wearable device connected with itself, but also the second wearable device connected with the external electronic device.

Effects obtainable in the present disclosure are not limited to the effects mentioned above, and other effects not mentioned will be clearly understood by those having ordinary knowledge in the art to which the present disclosure belongs from the description below.

### [Description of the Drawings]

FIG. 1 is a block diagram of an electronic device in a network environment according to various embodiments.
FIG. 2 illustrates an environment including an electronic device according to various embodiments.
FIG. 3 is a simplified block diagram of an electronic device according to various embodiments.
FIG. 4 is a simplified block diagram of a first wearable device according to various embodiments.
FIG. 5 illustrates an example of an external shape of a first wearable device according to various embodiments.
FIG. 6 illustrates an example of a mode of a first wearable device according to various embodiments.
FIG. 7 is a flowchart illustrating an operation of an electronic device according to various embodiments.
FIG. 8 is another flowchart illustrating an operation of an electronic device according to various embodiments.
FIG. 9 illustrates an example of an operation of an electronic device according to various embodiments.
FIG. 10 illustrates another example of an operation of an electronic device according to various embodiments.
FIG. 11 is another flowchart illustrating an operation of an electronic device according to various embodiments.
FIG. 12 is another flowchart illustrating an operation of an electronic device according to various embodiments.
FIG. 13 illustrates another example of an operation of an electronic device according to various embodiments.
FIG. 14 illustrates another example of an operation of an electronic device according to various embodiments.
FIG. 15 is another flowchart illustrating an operation of an electronic device according to various embodiments.

### [Mode for Invention]

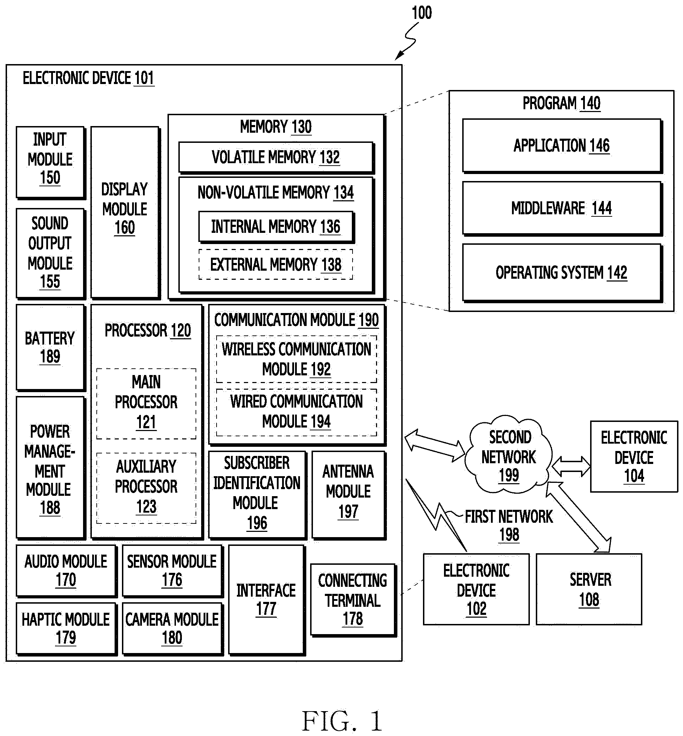

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

According to an embodiment, a processor (e.g., the processor 120 of FIG. 1) of an electronic device (e.g., the electronic device 101 of FIG. 1) may establish a connection with a first wearable device worn by a first user of the electronic device. The processor may establish a connection with an external electronic device connected with a second wearable device worn by a second user. The processor may control the first wearable device and the second wearable device together. For example, the first wearable device and the second wearable device may include a gait enhancing and motivating system (GEMS) to assist a user (e.g., the first user and the second user) in walking.

A detailed operation of the electronic device (or the processor of the electronic device) for the above-described embodiment may be described below. The electronic device described below may correspond to the electronic device 101 of FIG. 1.

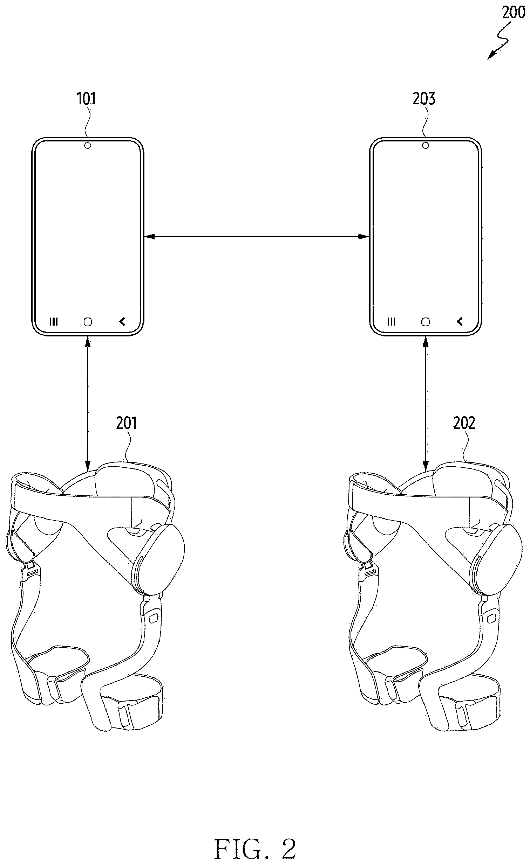

FIG. 2 illustrates an environment including an electronic device according to various embodiments.

Referring to FIG. 2, an environment 200 may include an electronic device 101, a first wearable device 201, a second wearable device 202, and an external electronic device 203.

For example, the electronic device 101 and the first wearable device 201 may be used by a first user. For example, the first wearable device 201 may be operated while worn by the first user. The electronic device 101 may establish a connection with the first wearable device 201. The electronic device 101 may transmit a request signal for controlling the first wearable device 201.

For example, the external electronic device 203 and the second wearable device 202 may be used by a second user. For example, the second wearable device 202 may be operated while worn by the second user. The external electronic device 203 may establish a connection with the second wearable device 202. The electronic device 101 may transmit a request signal for controlling the second wearable device 202.

According to an embodiment, the electronic device 101 may establish a connection with the external electronic device 203. The electronic device 101 may transmit the request signal for controlling the second wearable device 202 to the external electronic device 203. The external electronic device 203 may control the second wearable device 202 based on the request signal.

For example, the electronic device 101 may operate as a master device. The external electronic device 203 may operate as a slave device. While the electronic device 101 operates as the master device, the electronic device 101 may control the external electronic device 203 and/or the second wearable device 202 connected with the external electronic device 203.

According to an embodiment, the electronic device 101 may directly transmit the request signal for controlling the second wearable device 202 to the second wearable device 202. According to an embodiment, the electronic device 101 may transmit the request signal for controlling the second wearable device 202 to the first wearable device 201. In a state in which the first wearable device 201 establishes a connection with the second wearable device 202, the first wearable device 201 may transmit the request signal for controlling the second wearable device 202, received from the electronic device 101, to the second wearable device 202.

Hereinafter, a detailed configuration of the electronic device 101 and the first wearable device 201 may be described. According to an embodiment, the external electronic device 203 may correspond to the electronic device 101. According to an embodiment, the second wearable device 202 may correspond to the first wearable device 201.

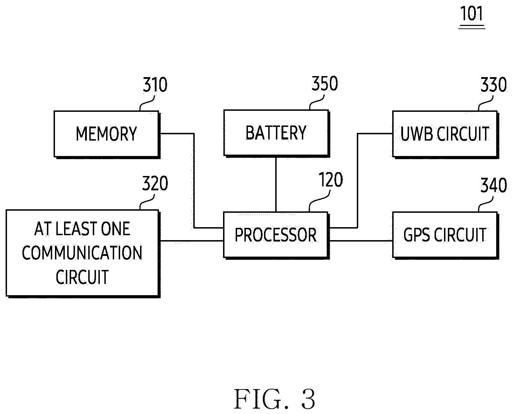

FIG. 3 is a simplified block diagram of an electronic device according to various embodiments.

Referring to FIG. 3, an electronic device 101 of FIG. 3 may correspond to the electronic device 101 of FIG. 1 and the electronic device 101 of FIG. 2. The electronic device 101 may include a processor 120, a memory 310, at least one communication circuit 320, a UWB circuit 330, a global positioning system (GPS) circuit 340, and/or a battery 350. According to an embodiment, the electronic device 101 may include at least one of the processor 120, the memory 310, the at least one communication circuit 320, the UWB circuit 330, the GPS circuit 340, and the battery 350. For example, at least a portion of the processor 120, the memory 310, the at least one communication circuit 320, the UWB circuit 330, the GPS circuit 340, and the battery 350 may be omitted according to an embodiment.

According to an embodiment, the processor 120 may control the memory 310, the at least one communication circuit 320, the UWB circuit 330, the GPS circuit 340, and the battery 350. The memory 310, the at least one communication circuit 320, the UWB circuit 330, the GPS circuit 340, and the battery 350 may be controlled by the processor 120. For example, the processor 120 may obtain information stored in the memory 310. The processor 120 may identify the information stored in the memory 310. For another example, the processor 120 may establish a connection with an external electronic device (e.g., the external electronic device 203 of FIG. 2) through the at least one communication circuit 320.

According to an embodiment, the memory 310 may store information within the electronic device 101. For example, the memory 310 may correspond to the memory 130 of FIG. 1. For example, the memory 310 may be a volatile memory unit or units. For example, the memory 310 may be a nonvolatile memory unit or units. For another example, the memory 310 may be another form of computer readable medium, such as a magnetic or optical disk.

According to an embodiment, the at least one communication circuit 320 and the UWB circuit 330 may each correspond to at least a portion of the communication module 190 of FIG. 1.

According to an embodiment, the at least one communication circuit 320 may be used for various radio access technology (RAT). For example, the at least one communication circuit 320 may be used to perform bluetooth communication or wireless local area network (WLAN) communication. For example, the processor 120 may establish a connection with the external electronic device through the at least one communication circuit 320. For another example, the processor 120 may establish a connection with a first wearable device through the at least one communication circuit 320.

According to an embodiment, the UWB circuit 330 may be used to transmit and/or receive a UWB signal. The UWB circuit 240 may be used to identify a distance between the electronic device 101 and the external electronic device (or an external object including the external electronic device). For example, the processor 120 may transmit the UWB signal through the UWB circuit 240. The UWB signal may be reflected to the external electronic device (or the external object including the external electronic device). The processor 120 may receive a reflection signal relative to the UWB signal. The processor 120 may identify the distance between the electronic device 101 and the external electronic device based on the reflection signal relative to the UWB signal.

According to an embodiment, the GPS circuit 340 may correspond to a GNSS communication module which is an example of the wireless communication module 192 of FIG. 1. The GPS circuit 340 may be used to receive a GPS signal. The GPS may include at least one of global navigation satellite system (GLONASS), Beidou Navigation Satellite System (hereinafter referred to as "beidou"), quasi-zenith satellite system (QZSS), indian reginal satellite system (IRNSS), or the european global satellite-based navigation system (Galileo), according to an area of use or bandwidth. The electronic device 101 may obtain data on a location of the electronic device 101 using the GPS circuit 340.

According to an embodiment, the battery 350 may be used to supply power to the electronic device 101. The battery 350 may include a rechargeable battery. The processor 120 may identify information on a remaining battery capacity of the battery 350. For example, the processor 120 may identify information on whether the remaining battery capacity of the battery 350 is less than or equal to a reference remaining battery capacity.

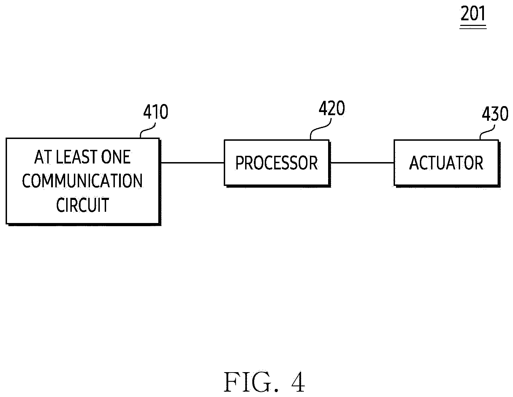

FIG. 4 is a simplified block diagram of a first wearable device according to various embodiments.

Referring to FIG. 4, a first wearable device 201 of FIG. 4 may correspond to the first wearable device 201 or the second wearable device 202 of FIG. 2. The first wearable device 201 may operate while worn by a user. For example, the first wearable device 201 may be used to assist or resist a walking of the user.

According to an embodiment, the first wearable device 201 may include at least one communication circuit 410, a processor 420, and/or an actuator 430. According to an embodiment, the first wearable device 201 may include at least one of the at least one communication circuit 410, the processor 420, and the actuator 430. For example, at least a portion of the at least one communication circuit 410, the processor 420, and the actuator 430 may be omitted according to an embodiment.

According to an embodiment, the processor 420 may control the at least one communication circuit 410 and the actuator 430. The at least one communication circuit 410 and the actuator 430 may be controlled by the processor 420. For example, the processor 420 may generate torque through the actuator 430.

According to an embodiment, the at least one communication circuit 410 may correspond to the at least one communication circuit 220 of FIG. 2.

According to an embodiment, the actuator 430 may be used to generate the torque. The actuator 430 may be used to output the torque. The processor 120 may assist or resist the walking of the user by adjusting the torque of the actuator 430. For example, the actuator 430 may generate resist torque. The actuator 430 may resist the walking of the user through the resist torque. For another example, the actuator 430 may generate assist torque. The actuator 430 may assist the walking of the user through the assist torque.

Although not illustrated, the first wearable device 201 may further include various circuits (or devices).

For example, the first wearable device 201 may further include an output device. For example, the output device may include a speaker or a light emitting diode (LED). The first wearable device 201 may provide a notification to the user of the first wearable device 201 through the output device.

For another example, the first wearable device 201 may further include at least one sensor. The at least one sensor may correspond to the sensor module 176 of FIG. 1. For example, the at least one sensor may include an acceleration sensor, a gyro sensor, or a magnetometer. The acceleration sensor may identify (or measure or detect) acceleration of the first wearable device 201 in three directions of the x-axis, y-axis, and z-axis. The gyro sensor may identify (or measure or detect) angular velocity of the first wearable device 201 in three directions of the x-axis, y-axis, and z-axis. The magnetometer may detect magnitude of a magnetic field. For an example, the magnetometer may identify that the first wearable device 201 is worn by the user based on a change in the magnitude of the magnetic field.

According to an embodiment, the first wearable device 201 may establish a connection with the electronic device (e.g., the electronic device 101 of FIG. 2) through the at least one communication circuit 410. For example, the first wearable device 201 may establish a connection with the electronic device through bluetooth communication.

According to an embodiment, the processor 420 of the first wearable device 201 may receive a signal for controlling the actuator 430 of the first wearable device 201 from the electronic device. For example, the processor 420 may receive a request signal from the electronic device to output torque having a value within a designated range. The processor 420 may output the torque having the value within the designated range through the actuator 430 in response to the request signal.

In FIG. 3, an electrically connected internal block diagram of the first wearable device 201 is illustrated, but in FIG. 4, an external shape of the first wearable device 201 may be illustrated.

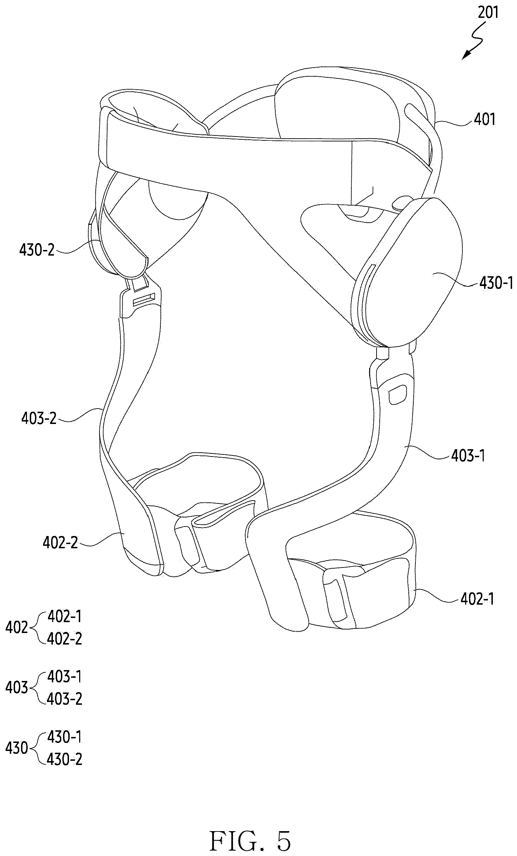

FIG. 5 illustrates an example of an external shape of a first wearable device according to various embodiments.

Referring to FIG. 5, a first wearable device 201 may include a first support part 401, a second support part 402, a connection part 403, and an actuator 430.

According to an embodiment, the first wearable device 201 may be formed to be worn on a user's lower body. For example, the first support part 401 may be fixed to the waist or hip of the user.

For example, the second support part 402 may include a second support part 402-1 and a second support part 402-2. The second support part 402 may be fixed to the leg (or thigh) of the user. For an example, the second support part 402-1 may be fixed to the left leg of the user. The second support part 402-2 may be fixed to the right leg of the user.

According to an embodiment, the actuator 430 may be attached to the first support part 401. For example, the actuator 430 may include an actuator 430-1 and an actuator 430-2. The actuator 430-1 may be attached to a first part (e.g., left side) of the first support part 401. The actuator 430-2 may be attached to a second part (e.g., right side) opposite to the first part of the first support part 401.

According to an embodiment, the connection part 403 may rotatably connect the second support part 402 relative to the first support part 401 through the actuator 430. For example, the connection part 403 may include a connection part 403-1 and a connection part 403-2. The connection part 403-1 may rotatably connect the second support part 402-1 relative to the first support part 401 through the actuator 430-1. The connection part 403-2 may rotatably connect the second support part 402-2 relative to the first support part 401 through the actuator 430-2.

According to an embodiment, the actuator 430 may generate torque to resist movement (e.g., walking) of the user. For example, the actuator 430 may generate resist torque. When the user lifts the leg, the actuator 430 may generate torque to generate a force in a direction opposite to a direction in which the user lifts the leg. When the user changes from a state in which the leg is raised to a state in which the leg is lowered, the actuator 430 may generate torque to generate a force in a direction opposite to a direction in which the leg is lowered. The above-described resist torque may not be a fixed value. For example, the resist torque may refer to a set of torques having a value within a range set to resist the movement of the user.

According to an embodiment, the actuator 430 may generate torque to assist the movement (e.g., walking) of the user. For example, the actuator 430 may generate assist torque. When the user lifts the leg, the actuator 430 may generate torque to generate a force in a direction corresponding to a direction in which the user lifts the leg. When the user changes from a state in which the leg is raised to a state in which the leg is lowered, the actuator 430 may generate torque to generate a force in a direction corresponding to a direction in which the user lowers the leg. The above-described assist torque may not be a fixed value. For example, the resist torque may refer to a set of torques having a value within a range set to assist the movement of the user.

According to an embodiment, the actuator 430-1 and the actuator 430-2 may output (or generate) torque in different ranges. For example, when the user of the first wearable device 201 walks on a sidewalk tilted to one side, the actuator 430-1 and the actuator 430-2 may assist the movement of the user by outputting the torque in different ranges.

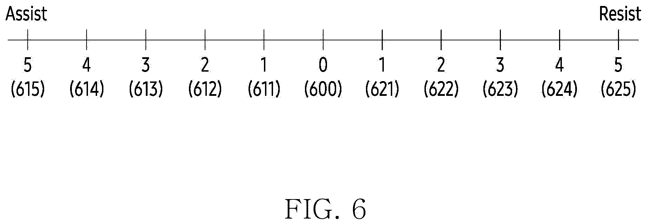

According to an embodiment, the first wearable device 201 may operate in a plurality of modes for assisting or resisting the movement of the user. A detailed example of the plurality of modes operating in the first wearable device 201 may be described with reference to FIG. 6.

FIG. 6 illustrates an example of a mode of a first wearable device according to various embodiments.

Referring to FIG. 6, a first wearable device 201 may operate in a plurality of modes to assist or resist movement of a user. For example, the plurality of modes in which the first wearable device 201 operates may include a plurality of modes for outputting assist torque and a plurality of modes for outputting resist torque. The plurality of modes in which the first wearable device 201 operates may include a mode that does not output torque. In each mode, the first wearable device 201 may output torque having values within different designated ranges.

For example, the resist torque and the assist torque may act in opposite directions. For an example, the resist torque may act in a direction to resist the movement of the user. The assist torque may act in a direction to assist the movement of the user.

The magnitude of the resist torque may have values that change according to a posture (e.g., a posture according to walking) of the user. The magnitude of the resist torque may have values that change according to the posture (e.g., the posture according to walking) of the user. For convenience of description, the magnitude of the resist torque may be described as corresponding to the magnitude of force to resist the movement of the user. The magnitude of the assist torque may be described as corresponding to the magnitude of the force to assist the movement of the user.

For example, the plurality of modes for outputting the assist torque may include an assist 1 mode 611 to an assist 5 mode 615. In the assist 5 mode 615, the magnitude of the assist torque may be set to be the greatest. In the assist 1 mode 611, the magnitude of the assist torque may be set to be the smallest. Since the mode is changed from the assist 5 mode 615 to the assist 1 mode 611, a processor 420 of the first wearable device 201 may reduce the magnitude of the assist torque. For example, a processor 620 may reduce the magnitude of the assist torque by changing a designated range since the mode is changed from the assist 5 mode 615 to the assist 1 mode 611. For an example, in the assist 1 mode 611, the processor 420 may output torque having values within a first designated range. In an assist 2 mode 612, the processor 420 may output torque having values within a second designated range. A first force to assist the movement of the user acting through the torque having values within the second designated range may be set to be greater than a second force to assist the movement of the user acting through the torque having values within the first designated range.

For example, in a zero mode 600, the processor 420 may not output torque. The zero mode 600 may be referred to as an assist 0 mode or a resist 0 mode.

For example, the plurality of modes for outputting resist torque may include a resist 1 mode 621 to a resist 5 mode 625. In the resist 5 mode 625, the magnitude of the resist torque may be set to be the greatest. In the resist 1 mode 621, the magnitude of the resist torque may be set to be the smallest.

Since the mode is changed from the resist 1 mode 621 to the resist 5 mode 625, the processor 420 may increase the magnitude of the resist torque. For example, the processor 420 may increase the magnitude of the resist torque by changing the designated range since the mode is changed from the resist 1 mode 621 to the resist 5 mode 625. For example, the processor 420 may increase the magnitude of the resist torque by changing the designated range since the mode is changed from the resist 1 mode 621 to the resist 5 mode 625. For an example, in the resist 1 mode 621, the processor 420 may output the torque having values within the first designated range. In the resist 2 mode 622, the processor 420 may output the torque having values within the second designated range. The first force to resist the movement of the user acting through the torque having values within the second designated range may be set to be greater than the second force to resist the movement of the user acting through the torque having values within the first designated range.

FIG. 6 illustrates an example of the plurality of modes of the first wearable device 201, but this is exemplary and is not limited thereto. Contrary to the above-described example, the mode of the first wearable device 201 may be variously set and may be referred to by various names.

For example, nine modes may be further included between the two adjacent modes described above. For an example, nine modes may be further included between a resist 4 mode 614 and a resist 3 mode 613. A resist 4.1 mode to a resist 4.9 mode may be included between the resist 4 mode 614 and the resist 3 mode 613.

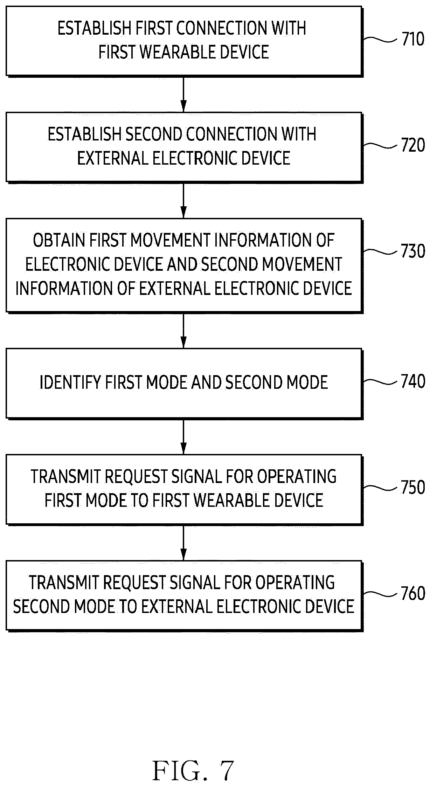

FIG. 7 is a flowchart illustrating an operation of an electronic device according to various embodiments. This method may be executed by the electronic device 101 and the processor 120 of the electronic device 101 illustrated in FIGS. 2 and 3.

Referring to FIG. 7, in operation 710, the processor 120 may establish a first connection with a first wearable device (e.g., the first wearable device 201 of FIG. 2).

According to an embodiment, the first wearable device may be worn by a first user of the electronic device 101. For example, the first wearable device may be used to assist or resist movement (e.g., walking) of the first user of the electronic device 101. For example, the first wearable device may include a first actuator. The first wearable device may be used to assist or resist the movement of the first user by generating torque having values within a designated range through the first actuator.

According to an embodiment, the processor 120 may establish the first connection with the first wearable device through bluetooth. For example, the first connection may be established based on bluetooth.

According to an embodiment, the processor 120 may identify that the first wearable device is worn by a user. For example, the processor 120 may receive information for indicating that the first wearable device is worn by the user (e.g., the first user), from the first wearable device. The processor 120 may identify that the first wearable device is worn by the user based on the information for indicating that the first wearable device is worn by the user. The processor 120 may perform a user authentication procedure based on identifying that the first wearable device is worn by the user. The processor 120 may identify the user wearing the first wearable device as the first user based on the user authentication procedure. After the user authentication procedure is performed, the processor 120 may identify that the first wearable device is worn by the first user.

In operation 720, the processor 120 may establish a second connection with an external electronic device. According to an embodiment, the external electronic device may correspond to the electronic device 101.

According to an embodiment, the external electronic device may be in a connected state with a second wearable device. For example, the external electronic device may control the second wearable device. The external electronic device may establish a connection corresponding to the first connection with the second wearable device. For an example, the external electronic device may establish a bluetooth connection with the second wearable device.

According to an embodiment, the second wearable device may be worn by a second user. For example, the second wearable device may be used to assist or resist the movement (e.g., walking) of the second user of the external electronic device. For example, the second wearable device may include a second actuator. The second wearable device may be used to assist or resist the movement of the second user by generating torque having values within a designated range through the second actuator.

According to an embodiment, the processor 120 may establish a second connection with the external electronic device through a wireless local area network. For example, the second connection may be established based on the wireless local area network.

In operation 730, the processor 120 may obtain first movement information of the electronic device 101 and second movement information of the external electronic device. For example, the processor 120 may obtain the first movement information of the electronic device 101 and the second movement information of the external electronic device while the first connection and the second connection are maintained.

According to an embodiment, the processor 120 may obtain data on locations of the electronic device 101 through a GPS circuit 240. The processor 120 may obtain the first movement information of the electronic device 101 based on the data on the locations of the electronic device 101 obtained through the GPS circuit 240. For example, the first movement information of the electronic device 101 may be obtained from the data on the locations of the electronic device 101 obtained through the GPS circuit 240.

According to an embodiment, the processor 120 may obtain the first movement information of the electronic device 101 through a UWB circuit 230. For example, the processor 120 may identify an external object around the electronic device 101 through the UWB circuit 230 and obtain the first movement information of the electronic device 101 based on a change in the location of the electronic device 101 relative to the identified external object.

According to an embodiment, the processor 120 may obtain the second movement information of the external electronic device from the external electronic device. The external electronic device may transmit the second movement information of the external electronic device to the electronic device 101. The processor 120 may receive the second movement information of the external electronic device from the external electronic device.

According to an embodiment, the processor 120 may identify a distance between the electronic device 101 and the external electronic device based on the first movement information of the electronic device 101 and the second movement information of the external electronic device. The processor 120 may identify that the distance between the electronic device 101 and the external electronic device is greater than or equal to a designated distance.

For example, the processor 120 may identify that the external electronic device moving in a first direction is followed by the electronic device 101 moving in the first direction based on the first movement information of the electronic device and the second movement information of the external electronic device. The processor 120 may identify that the distance between the electronic device 101 and the external electronic device that is followed by the electronic device 101 is greater than or equal to the designated distance.

According to an embodiment, the electronic device 101 may be in a state carried by the first user. The electronic device 101 may be carried by the first user. A location of the electronic device 101 may correspond to a location of the first user. A location of the first wearable device worn by the first user may correspond to the location of the first user. The location of the electronic device 101 may correspond to the location of the first wearable device.

According to an embodiment, the external electronic device may be in a state carried by the second user. The external electronic device may be carried by the second user. A location of the external electronic device may correspond to a location of the second user. A location of the second wearable device worn by the second user may correspond to the location of the second user. The location of the external electronic device may correspond to the location of the second wearable device.

In operation 740, the processor 120 may identify a first mode and a second mode. For example, based on the first movement information of the electronic device 101 and the second movement information of the external electronic device, the processor 120 may identify the first mode in which the first wearable device will operate using the first actuator and the second mode in which the second wearable device will operate using the second actuator.

According to an embodiment, the first mode may be set as one of a plurality of modes in which the first wearable device outputs assist torque acting in a direction to assist the movement of the first user using the first actuator.

The second mode may be set as one of a plurality of modes in which the second wearable device outputs resist torque acting in a direction to resist the movement of the second user using the second actuator. In the first mode, the magnitude of the torque (hereinafter referred to as a first torque) output using the first actuator may be changed based on the posture of the first user. In the second mode, the magnitude of the torque (hereinafter referred to as a second torque) output using the second actuator may be changed based on the posture of the second user.

For example, the first mode may include a mode in which the electronic device 101 outputs the first torque having values within a first designated range using the first actuator. The second mode may include a mode in which the external electronic device outputs the second torque having values within a second designated range using the second actuator.

For an example, the first torque having values within the first designated range may be outputted from the first wearable device using the first actuator. For example, the first wearable device may output the first torque having values within the first designated range using the first actuator. For an example, the second torque having values within the second designated range may be outputted from the second wearable device using the second actuator. For example, the second wearable device may output the second torque having values within the second designated range using a second actuator.

According to an embodiment, the processor 120 may identify that the distance between the electronic device 101 and the external electronic device is greater than or equal to the designated distance. The processor 120 may identify that the external electronic device moving in the first direction is followed by the electronic device 101 moving in the first direction. The processor 120 may identify the first mode to resist movement in the first direction and the second mode to assist movement in the first direction. For example, the processor 120 may identify the first mode to resist the movement in the first direction and the second mode to assist the movement in the first direction based on identifying that the external electronic device moving in the first direction is followed by the electronic device 101 moving in the first direction.

For example, a direction of the first torque output in the first mode to resist the movement in the first direction may be set opposite to a direction of the second torque output in the second mode to assist the movement in the first direction.

According to an embodiment, the processor 120 may identify torque that is caused by the first user and obtained from the first wearable device. The first wearable device may obtain torque caused by the walking of the first user. For example, the processor 120 may identify torque caused by the walking of the first user obtained from the first wearable device. For an example, the first wearable device may identify net torque based on an operation of the first actuator. The first wearable device may identify torque output through the first actuator. The first wearable device may identify torque caused by the walking of the first user based on the net torque and the torque output through the first actuator. The processor 120 may identify the first mode based on the torque caused by the walking of the first user.

According to an embodiment, the processor 120 may obtain operation record information of the first wearable device stored in the memory 310. The operation record of the first wearable device may be related to a use record of the first wearable device of the user. The processor 120 may identify the first mode based on the operation record information of the first wearable device.

In operation 750, the processor 120 may transmit a request signal for operating the first mode to the first wearable device. For example, the processor 120 may transmit a request signal for operating the first mode using the first actuator to the first wearable device through the first connection.

In operation 760, the processor 120 may transmit a request signal for operating the second mode to the external electronic device. For example, the processor 120 may transmit a request signal for operating the second mode using the second actuator to the external electronic device through the second connection. The external electronic device may control the second wearable device to operate in the second mode in response to the request signal for operating the second mode. For example, the external electronic device may transmit the request signal for operating the second mode received from the electronic device 101 to the second wearable device.

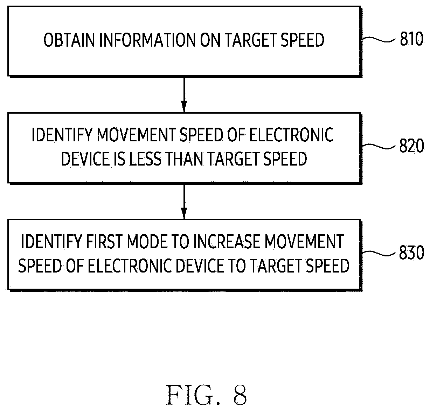

FIG. 8 is another flowchart illustrating an operation of an electronic device according to various embodiments. This method may be executed by the electronic device 101 and the processor 120 of the electronic device 101 illustrated in FIGS. 2 and 3.

Referring to FIG. 8, operations 810 to 830 may be related to the operations 730 to 740 of FIG. 7.

In operation 810, the processor 120 may obtain information on target speed. For example, the processor 120 may obtain information on the target speed based on a user input of a first user.

According to an embodiment, the processor 120 may obtain a user input from the first user. The processor 120 may obtain a user input from the first user based on establishing a second connection with an external electronic device. The user input may include an input for setting the target speed.

According to an embodiment, the target speed may be related to movement speed of the electronic device 101 and/or movement speed of the external electronic device. For example, the processor 120 may obtain a user input for setting the movement speed of the electronic device 101 to the target speed from the first user. The processor 120 may obtain information on the target speed based on the user input.

In operation 820, the processor 120 may identify that the movement speed of the electronic device 101 is less than the target speed. For example, the processor 120 may identify that the movement speed of the electronic device 101 obtained based on a first movement information of the electronic device 101 is less than the target speed.

According to an embodiment, the processor 120 may identify the movement speed of the electronic device 101 based on the first movement information of the electronic device 101. For example, the processor 120 may obtain the first movement information of the electronic device 101 based on data related to locations of the electronic device 101. For example, the processor 120 may identify a distance the electronic device 101 moved within a designated time interval. The processor 120 may identify the movement speed of the electronic device 101 based on identifying the distance the electronic device 101 moved within the designated time interval.

In operation 830, the processor 120 may identify the first mode to increase the movement speed of the electronic device 101 to the target speed.

According to an embodiment, the processor 120 may increase the movement speed of the electronic device 101 by controlling the first wearable device worn by the first user. The processor 120 may identify the first mode, which is a mode to operate in the first wearable device. For example, the processor 120 may identify the first mode to assist the movement of the electronic device 101 (or the movement of the first user).

Contrary to the operations 820 and 830, according to an embodiment, the processor 120 may identify that the movement speed of the electronic device 101 exceeds the target speed. The processor 120 may identify the first mode to reduce the movement speed of the electronic device 101 to the target speed. For example, the processor 120 may identify the first mode to resist the movement of the electronic device 101 (or the movement of the first user).

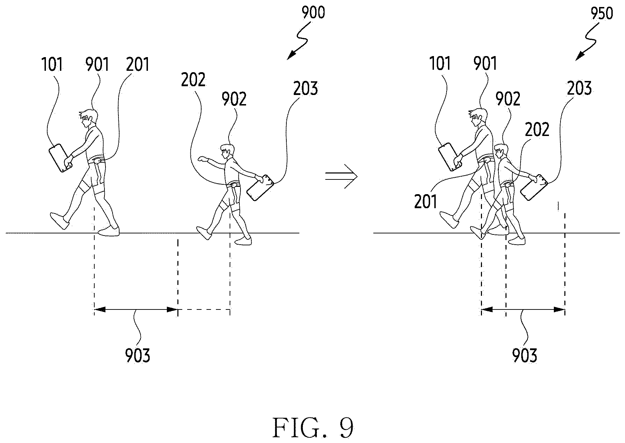

FIG. 9 illustrates an example of an operation of an electronic device according to various embodiments.

Referring to FIG. 9, an electronic device 101 may be carried by a first user 901. A first wearable device 201 may be worn by the first user 901. The electronic device 101 may be connected to the first wearable device 201. The electronic device 101 may control the first wearable device 201.

An external electronic device 203 may be carried by a second user 902. A second wearable device 202 may be worn by the second user 902. The external electronic device 203 may be connected to the second wearable device 202. The external electronic device 203 may control the second wearable device 202.

In state 900, a processor 120 of the electronic device 101 may identify that a distance between the electronic device 101 and the external electronic device 203 is greater than or equal to a designated distance 903.

For example, the processor 120 may obtain data related to a location of the electronic device 101 through a GPS circuit 340. The processor 120 may identify first movement information of the electronic device 101 based on the data related to the location of the electronic device 101.

For example, the processor 120 may obtain second movement information of the external electronic device 203 from the external electronic device 203. For an example, the processor 120 may receive the second movement information of the external electronic device 203 from the external electronic device 203.

According to an embodiment, the processor 120 may identify that the external electronic device 203 moving in a first direction is followed by the electronic device 101 moving in the first direction based on the first movement information of the electronic device 101 and the second movement information of the external electronic device 203.

For example, the processor 120 may identify that the electronic device 101 moves in the first direction, based on the first movement information of the electronic device 101. The processor 120 may identify that the external electronic device 203 also moves in the first direction based on the second movement information of the external electronic device 203. The processor 120 may identify that the electronic device 101 and the external electronic device 203 move in the first direction.

For example, the processor 120 may identify that the external electronic device is followed by the electronic device 101 based on the first movement information of the electronic device 101 and the second movement information of the external electronic device 203.

According to an embodiment, the processor 120 may identify the distance between the electronic device 101 and the external electronic device 203 based on the first movement information of the electronic device 101 and the second movement information of the external electronic device 203. The processor 120 may identify that the distance between the electronic device 101 and the external electronic device 203 is greater than or equal to the designated distance 903.

According to an embodiment, the processor 120 may identify the distance between the electronic device 101 and the external electronic device 203 through a UWB circuit 330. The processor 120 may transmit a UWB signal to the external electronic device 203 (or the second user 902) through the UWB circuit. The processor 120 may identify a reflection signal in which the UWB signal is reflected by the external electronic device 203 (or the second user 902). The processor 120 may identify the distance between the electronic device 101 and the external electronic device 203 based on the reflection signal.

Meanwhile, the processor 120 may identify a first mode in which the first wearable device will operate and a second mode in which the second wearable device will operate, based on identifying that the distance between the electronic device 101 and the external electronic device 203 is greater than or equal to the designated distance 903.

For example, the processor 120 may identify the first mode to resist movement in the first direction and the second mode to assist movement in the first direction. The processor 120 may transmit a request signal for operating the first mode to the first wearable device 201. The processor 120 may transmit a request signal for operating the second mode to the external electronic device 203. The external electronic device 203 may control the second wearable device 202 to operate in the second mode in response to the request signal for operating the second mode.

In state 950, after transmitting the request signal for operating the first mode and the request signal for operating the second mode, the processor 120 may identify that the distance between the electronic device 101 and the external electronic device 203 is changed to less than the designated distance 903. The processor 120 may change the first mode based on identifying that the distance between the electronic device 101 and the external electronic device 203 is changed to less than the designated distance 903. The processor 120 may change the second mode based on identifying that the distance between the electronic device 101 and the external electronic device 203 is changed to less than the designated distance 903. The processor 120 may change the mode in which the first wearable device operates and the mode in which the second wearable device operates so that the distance between the electronic device 101 and the external electronic device 203 is maintained less than the designated distance 903. For example, the processor 120 may change the mode in which the first wearable device will operate from the first mode to a third mode so that the distance between the electronic device 101 and the external electronic device 203 is maintained less than the designated distance 903. The processor 120 may change the mode in which the second wearable device will operate from the second mode to a fourth mode so that the distance between the electronic device 101 and the external electronic device 203 is maintained less than the designated distance 903. The processor 120 may transmit a request signal for operating the third mode to the first wearable device 201. The processor 120 may transmit a request signal for operating the fourth mode to the external electronic device 203.

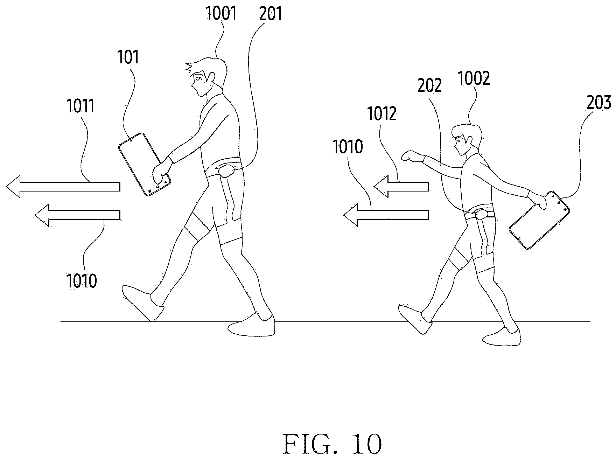

FIG. 10 illustrates another example of an operation of an electronic device according to various embodiments.

Referring to FIG. 10, a processor 120 of an electronic device 101 may obtain information on target speed 1010. For example, the processor 120 may obtain the information on the target speed 1010 based on a user input by a first user 1001. For another example, the processor 120 may obtain information on the first user 1001. The information on the first user 1001 may include information on physical ability of the first user 1001. The processor 120 may obtain information on a second user 1002. The processor 120 may receive information on the second user 1002 from an external electronic device 203. The information on the second user 1002 may include information on the physical ability of the second user 1002. The processor 120 may obtain information on the target speed 1010 based on the information on the first user 1001 and the information on the second user 1002.

The processor 120 may obtain data related to a location of the electronic device 101 through a GPS circuit 340. The processor 120 may identify first movement information of the electronic device 101 based on the data related to the location of the electronic device 101. The processor 120 may identify movement speed 1011 of the electronic device 101 based on the first movement information of the electronic device 101.

The processor 120 may obtain second movement information of the external electronic device 203 from the external electronic device 203. For an example, the processor 120 may receive the second movement information of the external electronic device 203 from the external electronic device 203. The processor 120 may identify movement speed 1012 of the external electronic device 203 based on the second movement information of the external electronic device 203.

The processor 120 may identify that the movement speed 1011 of the electronic device 101 is greater than or equal to the target speed 1010. The processor 120 may identify that the magnitude of the movement speed 1011 of the electronic device 101 is greater than or equal to the magnitude of the target speed 1010. The processor 120 may identify that the movement speed 1012 of the external electronic device 203 is less than the target speed 1010. The processor 120 may identify that the magnitude of the movement speed 1012 of the external electronic device 203 is less than the magnitude of the target speed 1010.

The processor 120 may identify a first mode to reduce the movement speed 1011 (or the magnitude of the movement speed 1011) of the electronic device 101 to the target speed 1010 (or the magnitude of the target speed 1010). The processor 120 may identify a second mode to increase the movement speed 1012 (or the magnitude of the movement speed 1012) of the external electronic device 203 to the target speed 1010 (or the magnitude of the target speed 1010).

For example, the processor 120 may identify the first mode to resist movement in a direction in which the electronic device 101 moves and the second mode to assist movement in a direction in which the external electronic device 203 moves. The processor 120 may transmit a request signal for operating the first mode to a first wearable device 201. The processor 120 may transmit a request signal for operating the second mode to the external electronic device 203. The external electronic device 203 may control a second wearable device 202 to operate in the second mode in response to the request signal for operating the second mode.

After transmitting the request signal for operating the first mode and the request signal for operating the second mode, the processor 120 may identify that the movement speed 1011 of the electronic device 101 corresponds to the target speed 1010. The processor 120 may identify that the movement speed 1012 of the external electronic device 203 corresponds to the target speed 1010.

The processor 120 may change the first mode based on identifying that the movement speed 1011 of the electronic device 101 corresponds to the target speed 1010. The processor 120 may change the second mode based on identifying that the movement speed 1012 of the external electronic device 203 corresponds to the target speed 1010. The processor 120 may change the mode in which the first wearable device operates and the mode in which the second wearable device operates to maintain the movement speed 1011 corresponding to the target speed 1010 and the movement speed 1012 corresponding to the target speed 1010. For example, the processor 120 may change the mode in which the first wearable device will operate from the first mode to a third mode so that the movement speed 1011 corresponding to the target speed 1010 and the movement speed 1012 corresponding to the target speed 1010 are maintained. The processor 120 may change the mode in which the second wearable device will operate from the second mode to a fourth mode so that the movement speed 1011 corresponding to the target speed 1010 and the movement speed 1012 corresponding to the target speed 1010 are maintained. The processor 120 may transmit a request signal for operating the third mode to the first wearable device 201. The processor 120 may transmit a request signal for operating the fourth mode to the external electronic device 203.

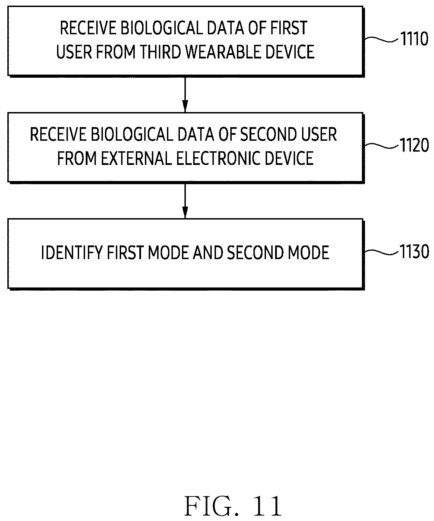

FIG. 11 is another flowchart illustrating an operation of an electronic device according to various embodiments. This method may be executed by the electronic device 101 and the processor 120 of the electronic device 101 illustrated in FIGS. 2 and 3.

Referring to FIG. 11, in operation 1110, the processor 120 may receive biological data of a first user from a third wearable device. For example, the third wearable device may include at least one sensor for obtaining the biological data of the first user. The third wearable device may obtain the biological data of the first user through the at least one sensor. The processor 120 may receive the biological data of the first user from the third wearable device.

For an example, the third wearable device may include a heart rate monitor (HRM) sensor. The third wearable device may obtain data on a heart rate of the first user through the HRM sensor. The third wearable device may transmit the data on the heart rate of the first user to the electronic device 101. The processor 120 may receive the data on the heart rate of the first user from the third wearable device.

In operation 1120, the processor 120 may receive biological data of a second user from an external electronic device. For example, the external electronic device may transmit the biological data of the second user obtained from a fourth wearable device to the electronic device 101. The processor 120 may receive the biological data of the second user from the external electronic device. For an example, the biological data of the second user may include data on the heart rate of the second user.

In operation 1130, the processor 120 may identify a first mode and a second mode. For example, the processor 120 may identify the first mode and the second mode based on the biological data of the first user and the biological data of the second user.

For example, the processor 120 may identify the data on the heart rate of the first user. The processor 120 may identify that the heart rate of the first user exceeds a designated value. The processor 120 may identify the first mode based on identifying that the heart rate of the first user exceeds the designated value.

The processor 120 may identify the data on the heart rate of the second user. The processor 120 may identify that the heart rate of the second user is less than or equal to the designated value. The processor 120 may identify the second mode based on identifying that the heart rate of the second user is less than or equal to the designated value.

For an example, the processor 120 may reduce the magnitude of resist torque output through a first wearable device based on the identified first mode. The processor 120 may reduce the amount of resistance(magnitude of resistance) caused to the first user through the first wearable device based on the identified first mode. The processor 120 may increase the magnitude of resist torque output through a second wearable device based on the identified second mode. The processor 120 may increase the amount of resistance caused to the second user through the second wearable device based on the identified second mode.

For another example, the processor 120 may change the torque output through the first wearable device from resist torque to assist torque based on the identified first mode. The processor 120 may change the torque output through the second wearable device from assist torque to resist torque based on the identified second mode.

For another example, the processor 120 may increase the magnitude of the assist torque output through the first wearable device based on the identified first mode. The processor 120 may increase the amount of assistance(magnitude of assistance) caused to the first user through the first wearable device based on the first mode. The processor 120 may reduce the magnitude of the assist torque output through the second wearable device based on the identified second mode. The processor 120 may reduce the amount of assistance caused to the second user through the second wearable device based on the second mode.

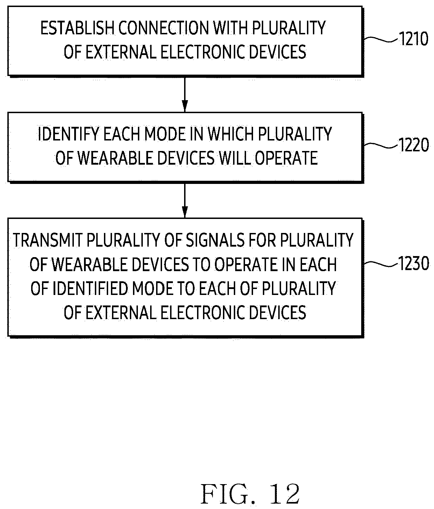

FIG. 12 is another flowchart illustrating an operation of an electronic device according to various embodiments. This method may be executed by the electronic device 101 and the processor 120 of the electronic device 101 illustrated in FIGS. 2 and 3.

Referring to FIG. 12, in operation 1210, the processor 120 may establish a connection with a plurality of external electronic devices. For example, while the electronic device 101 operates as a master device, the processor 120 may establish a connection with the plurality of external electronic devices that operate as slave devices and are located within a designated distance from the electronic device 101.

For example, the electronic device 101 may be connected to a first wearable device. Each of the plurality of external electronic devices may be connected to each of a plurality of wearable devices. For example, the plurality of external electronic devices may include a first external electronic device to a third external electronic device. The plurality of wearable devices may include a second wearable device to a fourth wearable device. For an example, the first external electronic device may be connected to the second wearable device. A second external electronic device may be connected to a third wearable device. The third external electronic device may be connected to the fourth wearable device.

According to an embodiment, the plurality of external electronic devices may be located within a designated distance from the electronic device 101. For example, the plurality of external electronic devices may be located within a distance in which a connection with the electronic device 101 may be established.

According to an embodiment, the electronic device 101 may operate as a master device. The plurality of external electronic devices may operate as slave devices. For example, while the electronic device 101 operates as the master device, the processor 120 of the electronic device 101 may control the plurality of external electronic devices and/or the plurality of wearable devices. The processor 120 may control the plurality of external electronic devices and/or the plurality of wearable devices by transmitting a signal (or a request signal) to the plurality of external electronic devices.

In operation 1220, the processor 120 may identify each mode in which the plurality of wearable devices will operate.

According to an embodiment, the processor 120 may receive information on movement of the plurality of external electronic devices from each of the plurality of external electronic devices. The processor 120 may identify each mode in which the plurality of wearable devices will operate so that the plurality of external electronic devices move based on a designated interval. For example, the processor 120 may identify each mode in which the plurality of wearable devices will operate so that the plurality of external electronic devices move in a line at the designated interval.

According to an embodiment, the processor 120 may identify a mode in which the first wearable device will operate so that the electronic device 101 also moves together with the plurality of external electronic devices based on the designated interval.

According to an embodiment, the processor 120 may receive information on the user of each of the plurality of external electronic devices from the plurality of external electronic devices. The processor 120 may obtain information on target speed based on information on the user of each of the plurality of external electronic devices and information on a first user of the electronic device 101.

For example, the processor 120 may receive information (or operation record information) on a walking record of each user of the plurality of external electronic devices. The processor 120 may obtain information on a walking record of the first user. The processor 120 may obtain information on the target speed based on information on the walking record of the first user and the walking record of each user of the plurality of external electronic devices. The processor 120 may identify each mode in which the plurality of external electronic devices will operate based on the information on the target speed.

For another example, the processor 120 may receive information on walking speed of each user of the plurality of external electronic devices (or information on movement speed of each of the plurality of external electronic devices). The processor 120 may obtain information on the walking speed of the first user (or information on the movement speed of the electronic device). The processor 120 may obtain information on the target speed based on information on the walking speed of each user of the plurality of external electronic devices or information on the walking speed of the first user. The processor 120 may identify each mode in which the plurality of external electronic devices will operate based on information on the target speed.

For another example, the processor 120 may identify information on torque caused by the walking of the first user, which is obtained from the first wearable device. The processor 120 may identify information on torque caused by the walking of each of the plurality of users, which is obtained from the plurality of wearable devices. The processor 120 may obtain the information on the target speed based on information on the torque caused by the walking of the first user and the information on the torque caused by the walking of each of the plurality of users. The processor 120 may identify each mode in which the plurality of external electronic devices will operate based on information on the target speed.

In operation 1230, the processor 120 may transmit a plurality of signals for operating the plurality of wearable devices in each identified mode to each of the plurality of external electronic devices. According to an embodiment, the processor 120 may transmit a signal for the first wearable device to operate the identified mode to the first wearable device.

According to an embodiment, the processor 120 may receive a user input for changing the target speed from the first user. The processor 120 may change each mode in which the plurality of wearable devices will operate based on the user input. The processor 120 may transmit a plurality of different signals for operating in each changed mode to each of the plurality of external electronic devices.

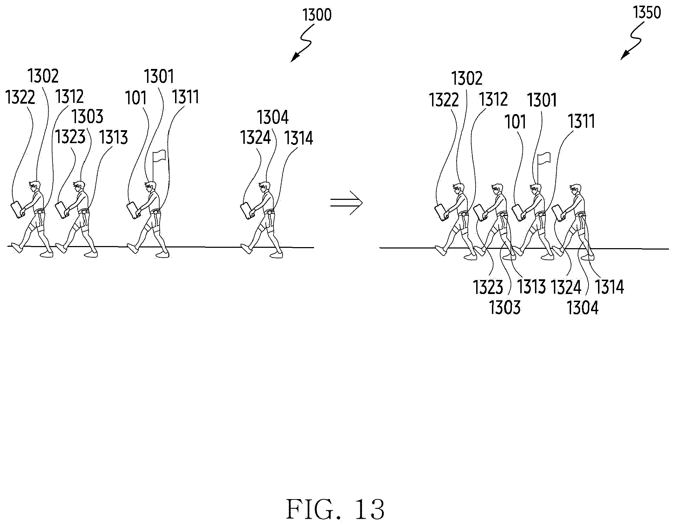

FIG. 13 illustrates another example of an operation of an electronic device according to various embodiments.

Referring to FIG. 13, an electronic device 101 may establish a connection with a plurality of external electronic devices. For example, the plurality of external electronic devices may include a first external electronic device 1322, a second external electronic device 1323, and a third external electronic device 1324.

The electronic device 101 may be used by a first user 1301. For an example, the electronic device 101 may be carried by the first user 1301. The first external electronic device 1322 may be used by a second user 1302. For an example, the electronic device 101 may be carried by the second user 1302. The second external electronic device 1323 may be used by a third user 1303. For an example, the electronic device 101 may be carried by the third user 1303.

The electronic device 101 may be connected to a first wearable device 1311. The first external electronic device 1322 may be connected to a second wearable device 1312. The second external electronic device 1323 may be connected to a third wearable device 1313. The third external electronic device 1324 may be connected to a fourth wearable device 1314.

The first wearable device 1311 may be worn by the first user 1301. The second wearable device 1312 may be worn by the second user 1302. The third wearable device 1313 may be worn by the third user 1303. The fourth wearable device 1314 may be worn by a fourth user 1304.

According to an embodiment, the electronic device 101 may operate as a master device. The first external electronic device 1322, the second external electronic device 1323, and the third external electronic device 1324 may operate as a slave device.

For example, a processor 120 of the electronic device 101 may form the electronic device 101, the first external electronic device 1322, the second external electronic device 1323, and the third external electronic device 1324 into one group. The processor 120 may identify each mode in which the first wearable device 1311 to the fourth wearable device 1314 will operate, so that the electronic device 101, the first external electronic device 1322, the second external electronic device 1323, and the third external electronic device 1324 may be moved at a designated interval. For an example, the processor 120 may identify a first mode in which the first wearable device 1311 will operate. The processor 120 may identify a second mode in which the second wearable device 1312 will operate. The processor 120 may identify a third mode in which the third wearable device 1313 will operate. The processor 120 may identify a fourth mode in which the fourth wearable device 1314 will operate.

According to an embodiment, the electronic device 101, the first external electronic device 1322, the second external electronic device 1323, and the third external electronic device 1324 may move in order of the first external electronic device 1322, the second external electronic device 1323, the electronic device 101, and the third external electronic device 1324. The processor 120 may identify whether the electronic device 101, the first external electronic device 1322, the second external electronic device 1323, and the third external electronic device 1324 move while maintaining the designated interval.

In state 1300, the processor 120 may identify that the electronic device 101, the first external electronic device 1322, the second external electronic device 1323, and the third external electronic device 1324 move without maintaining the designated interval. The processor 120 may identify each mode in which the first wearable device 1311 to the fourth wearable device 1314 will operate so that the electronic device 101, the first external electronic device 1322, the second external electronic device 1323, and the third external electronic device 1324 may be moved at the designated interval.

For an example, each torque to be outputted from the first wearable device 1311 to the third wearable device 1313 may be identified as resist torque. The torque to be outputted from the fourth wearable device 1314 may be identified as assist torque.

For an example, the processor 120 may set the magnitude of the resist torque to be outputted from the second wearable device 1312 worn by the second user 1302 located at the forefront to the largest. The processor 120 may set the magnitude of the resist torque to be outputted from the fourth wearable device 1314 worn by the fourth user 1304 located at the rearmost to the smallest.

According to an embodiment, the processor 120 may identify torque caused by a walking of the first user 1301 to the fourth user 1304. For example, the first wearable device 1311 may identify torque caused by the walking of the first user 1301. The second wearable device 1312 may identify torque caused by the walking of the second user 1302. The third wearable device 1313 may identify torque caused by the walking of the third user 1303. The fourth wearable device 1314 may identify torque caused by the walking of the fourth user 1304.

Based on each torque caused by the walking of the first user 1301 to the fourth user 1304, the processor 120 may identify each mode in which the first wearable device 1311 to the fourth wearable device 1314 will operate so that the electronic device 101, the first external electronic device 1322, the second external electronic device 1323, and the third external electronic device 1324 may be moved at the designated interval.

Meanwhile, the processor 120 may transmit a plurality of signals for operating the first wearable device 1311 to the fourth wearable device 1314 in each identified mode to each of the first wearable device 1311 to the fourth wearable device 1314. For example, the processor 120 may transmit a signal for operating the first wearable device 1311 the identified mode to the first wearable device 1311. The processor 120 may transmit a signal for operating the second wearable device 1312 the identified mode to the first external electronic device 1322. The processor 120 may transmit a signal for operating the third wearable device 1313 the identified mode to the second external electronic device 1323. The processor 120 may transmit a signal for operating the third wearable device 1314 the identified mode to the third external electronic device 1324.

In state 1350, after transmitting the plurality of signals for operating the first wearable device 1311 to the fourth wearable device 1314 in each identified mode to each of the first wearable device 1311 to the fourth wearable device 1314, the processor 120 may identify that the electronic device 101, the first external electronic device 1322, the second external electronic device 1323, and the third external electronic device 1324 move in the order of the first external electronic device 1322, the second external electronic device 1323, the electronic device 101, and the third external electronic device 1324. After transmitting the plurality of signals for operating the first wearable device 1311 to the fourth wearable device 1314 in each identified mode to each of the first wearable device 1311 to the fourth wearable device 1314, the processor 120 may identify that the electronic device 101, the first external electronic device 1322, the second external electronic device 1323, and the third external electronic device 1324 are moving while maintaining the designated interval.

The processor 120 may periodically change a mode for the electronic device 101, the first external electronic device 1322, the second external electronic device 1323, and the third external electronic device 1324 to move while maintaining the designated interval. The processor 120 may transmit a plurality of signals for operating the changed mode to the first wearable device 1311 to the fourth wearable device 1314. For example, as time passes, a state of the first user 1301 to the fourth user 1304 may be changed. Accordingly, the processor 120 may periodically identify and change the mode in which the first wearable device 1311 to the fourth wearable device 1314 operate in order to control the electronic device 101, the first external electronic device 1322, the second external electronic device 1323, and the third external electronic device 1324 to move while maintaining the designated interval.

Contrary to the above-described embodiment, according to an embodiment, the electronic device 101, the first external electronic device 1322, the second external electronic device 1323, and the third external electronic device 1324 may be connected through a server. The processor 120 of the electronic device 101 may transmit a signal for controlling the second wearable device 1312 to the fourth wearable device 1314 to the server. The server may transmit the received signal to the first external electronic device 1322 to the third external electronic device 1324. The processor may transmit a signal for controlling the second wearable device 1312 to the fourth wearable device 1314 to the first external electronic device 1322, the second external electronic device 1323, and the third external electronic device 1324 through the server, even in case that the electronic device 101, the first external electronic device 1322, the second external electronic device 1323, and the third external electronic device 1324 are located in different regions, respectively.

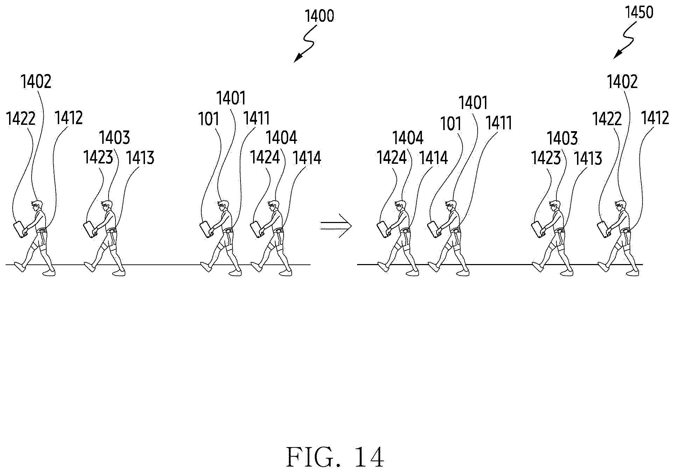

FIG. 14 illustrates another example of an operation of an electronic device according to various embodiments.

Referring to FIG. 14, an electronic device 101 may establish a connection with a plurality of external electronic devices. For example, the plurality of external electronic devices may include a first external electronic device 1422, a second external electronic device 1423, and a third external electronic device 1424.

The electronic device 101 may be used by a first user 1401. For an example, the electronic device 101 may be carried by the first user 1401. The first external electronic device 1422 may be used by a second user 1402. For an example, the electronic device 101 may be carried by the second user 1402. The second external electronic device 1423 may be used by a third user 1403. For an example, the electronic device 101 may be carried by the third user 1403.

The electronic device 101 may be connected to a first wearable device 1411. The first external electronic device 1422 may be connected to a second wearable device 1412. The second external electronic device 1423 may be connected to a third wearable device 1413. The third external electronic device 1424 may be connected to a fourth wearable device 1414.

The first wearable device 1411 may be worn by the first user 1401. The second wearable device 1412 may be worn by the second user 1402. The third wearable device 1413 may be worn by the third user 1403. The fourth wearable device 1414 may be worn by a fourth user 1404.

According to an embodiment, the electronic device 101 may operate as a master device. The first external electronic device 1422, the second external electronic device 1423, and the third external electronic device 1424 may operate as a slave device.

In state 1400, the electronic device 101, the first external electronic device 1422, the second external electronic device 1423, and the third external electronic device 1424 may move in order of the first external electronic device 1422, the second external electronic device 1423, the electronic device 101, and the third external electronic device 1424.

The processor 120 may change movement order of the electronic device 101, the first external electronic device 1422, the second external electronic device 1423, and the third external electronic device 1424. The processor 120 may identify each mode in which the first wearable device 1411 to the fourth wearable device 1414 will operate such that the electronic device 101, the first external electronic device 1422, the second external electronic device 1423, and the third external electronic device 1424 move in order of the third external electronic device 1424, the electronic device 101, the second external electronic device 1423, and the first external electronic device 1422. The processor 120 may transmit a signal for the first wearable device 1411 to operate the identified mode to the first wearable device 1411. The processor 120 may transmit a signal for the second wearable device 1412 to operate the identified mode to the first external electronic device 1422. The processor 120 may transmit a signal for the third wearable device 1413 to operate the identified mode to the second external electronic device 1423. The processor 120 may transmit a signal for the fourth wearable device 1414 to operate the identified mode to the third external electronic device 1424.

In state 1450, after a signal for operating the first wearable device 1411 to the fourth wearable device 1414 in each identified mode is transmitted, the electronic device 101, the first external electronic device 1422, the second external electronic device 1423, and the third external electronic device 1424 may move in the order of the third external electronic device 1424, the electronic device 101, the second external electronic device 1423, and the first external electronic device 1422.

In FIG. 14, an embodiment is illustrated in which the processor 120 changes the movement order of the plurality of external electronic devices (e.g., the first external electronic device 1422 to the third external electronic device 1424) and the electronic device 101 to reverse order, but is not limited thereto. The processor 120 may control the movement of the plurality of external electronic devices and the electronic device 101 by variously changing each mode in which the first wearable device 1411 to the fourth wearable device 1414 will operate. The processor 120 may control the movement of the plurality of external electronic devices and the electronic device 101 by assisting or resisting the movement of the first user to the fourth user.

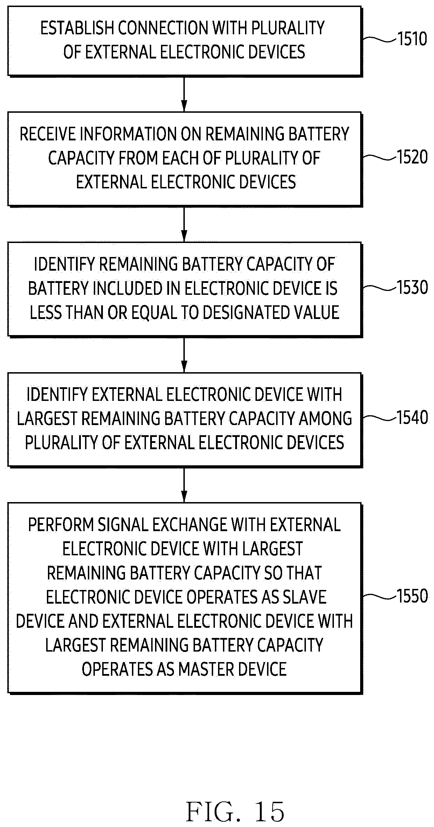

FIG. 15 is another flowchart illustrating an operation of an electronic device according to various embodiments. This method may be executed by an electronic device 101 and a processor 120 of the electronic device 101 illustrated in FIGS. 2 and 3.

Referring to FIG. 15, in operation 1510, the processor 120 may establish a connection with a plurality of external electronic devices. The operation 1510 may correspond to the operation 1110 of FIG. 11. For example, the electronic device 101 may operate as a master device. The plurality of external electronic devices may operate as a slave device.

In operation 1520, the processor 120 may receive information on remaining battery capacity from each of the plurality of external electronic devices. For example, while the electronic device 101 operates as the master device, the processor 120 may receive the information on the remaining battery capacity from each of the plurality of external electronic devices.

In operation 1530, the processor 120 may identify that remaining battery capacity of the battery included in the electronic device 101 is less than or equal to a designated value. For example, while the electronic device 101 operates as the master device, the processor 120 may identify that the remaining battery capacity of the battery included in the electronic device 101 is less than or equal to the designated value.

In operation 1540, the processor 120 may identify an external electronic device with the largest remaining battery capacity among the plurality of external electronic devices. For example, the processor 120 may identify the external electronic device with the largest remaining battery capacity among the plurality of external electronic devices based on identifying that the remaining battery capacity included in the electronic device 101 is less than or equal to the designated value.

In operation 1550, the processor 120 may perform signal exchange with the external electronic device with the largest remaining battery capacity so that the electronic device 101 operates as the slave device and the external electronic device with the largest remaining battery capacity operates as the master device.

The processor 120 may set the external electronic device with the largest remaining battery capacity as the master device based on completing the signal exchange. The processor 120 may set the electronic device 101 as the slave device.

In the above-described various embodiments, embodiments in which the processor 120 of the electronic device 101 controls the first wearable device and/or the second wearable device have been described, but it is not limited thereto. According to an embodiment, the first wearable device may operate independently of the electronic device 101. According to an embodiment, the first wearable device may perform a portion or all of the operations of the electronic device 101 according to the above-described embodiments.

According to various embodiments, an electronic device may comprise at least one memory configured to store instructions, global positioning system (GPS) circuit, at least one communication circuit, and at least one processor operably coupled to the at least one memory, the GPS circuit, and the at least one communication circuit, wherein the at least one processor may be configured, when executing the instructions, to establish a first connection with a first wearable device which comprises a first actuator and is worn by a first user of the electronic device, to establish a second connection with an external electronic device connected with a second wearable device which comprises a second actuator and is worn by a second user, while the first connection and the second connection are maintained, to obtain first movement information of the electronic device and second movement information of the external electronic device, based on the first movement information and the second movement information, to identify a first mode in which the first wearable device operates using the first actuator and a second mode in which the second wearable device operates using the second actuator, to transmit, through the first connection, a request signal for operating the first mode to the first wearable device, and to transmit, through the second connection, a request signal for operating the second mode to the external electronic device.

According to an embodiment, the first mode may comprise a mode for the electronic device to output first torque with values within a first designated range using the first actuator, and the second mode may comprise a mode for the external electronic device to output second torque with values within a second designated range using the second actuator.

According to an embodiment, the first movement information may be obtained from data related to locations of the electronic device obtained by the GPS circuit, wherein the second movement information may be obtained from the external electronic device, and wherein the processor may be configured, when executing the instructions, based on the first movement information and the second movement information, to identify a distance between the electronic device and the external electronic device, to identify that the distance between the electronic device and the external electronic device is greater than or equal to a designated distance, and based on identifying that the distance between the electronic device and the external electronic device is greater than or equal to the designated distance, to identify the first mode and the second mode.

According to an embodiment, the processor may be configured, when executing the instructions, to identify, based on the first movement information and the second movement information, that the external electronic device moving in a first direction is followed by the electronic device moving in the first direction.

According to an embodiment, the processor may be configured, when executing the instructions, to identify, based on identifying that the external electronic device moving in the first direction is followed by the electronic device moving in the first direction, identify the first mode for resisting movement in the first direction and the second mode for assisting movement in the first direction.

According to an embodiment, the direction of the first torque to resist the movement in the first direction may be set opposite to the direction of the second torque to assist the movement in the first direction.

According to an embodiment, the processor may be configured, when executing the instructions, to receive biological data of the first user from a third wearable device connected with the electronic device, to receive biological data of the second user from the external electronic device, and to identify, based on the biological data of the first user and the biological data of the second user, the first mode and the second mode.

According to an embodiment, the processor may be configured, when executing the instructions, to obtain, based on a user input of the first user, information on target speed, to identify movement speed of the electronic device identified based on the first movement information is less than the target speed, and based on identifying that movement speed of the electronic device obtained based on the first movement information is less than the target speed, to identify the first mode to increase the movement speed of the electronic device to the target speed.

According to an embodiment, the processor may be further configured, when executing the instructions, based on the first movement information and a mode of the first wearable device set before transmitting the request signal for operating the first mode, to identify torque caused by walking of the first user, and based on the torque caused by the walking of the first user, to identify the first mode.

According to an embodiment, the processor may be further configured, when executing the instructions, to establish connections with a plurality of external electronic devices operating as slave devices, located within a designated distance from the electronic device while the electronic device is operated as a master device, wherein each of the plurality of external electronic devices may be connected with each of a plurality of wearable devices.

According to an embodiment, the processor may be configured, when executing the instructions, to receive, from each of the plurality of external electronic devices, movement information of the plurality of external electronic devices, to identify each mode in which the plurality of wearable devices will operate so that the plurality of external electronic devices move based on a designated interval, and to transmit a plurality of signals for the plurality of wearable devices to operate in each of the identified mode to each of the plurality of external electronic devices.

According to an embodiment, the processor may be configured, when executing the instructions, to receive information on a user of each of the plurality of external electronic devices from the plurality of external electronic devices, to obtain information on a target speed based on the information on the user of each of the plurality of external electronic devices and information on the first user, based on the information on the target speed, to identify each mode in which the plurality of external electronic devices to be operated, to transmit a plurality of signals for the plurality of wearable devices to operate in each of the identified mode to each of the plurality of external electronic devices.

According to an embodiment, the processor may be configured, when executing the instructions, to obtain a user input for changing the target speed from the first user, and based on the user input, to change each mode in which the plurality of wearable devices will operate.

According to an embodiment, the electronic device may further comprise a rechargeable battery, and wherein the processor may be configured, when executing the instructions, while the electronic device operates as a master device, to receive information on remaining battery capacity from each of the plurality of external electronic devices, while the electronic device operates as a master device, to identify that remaining battery capacity of the battery comprised in the electronic device is less than or equal to a designated value, based on identifying that the remaining battery capacity of the battery comprised in the electronic device is less than or equal to the designated value, to identify an external electronic device with the largest remaining battery capacity among the plurality of external electronic devices, and in response to identifying the external electronic device with the largest remaining battery capacity, to perform signal exchange with the external electronic device with the largest remaining battery capacity so that the electronic device operates as a slave device and the external electronic device with the largest remaining battery capacity operates as a master device.

According to an embodiment, the electronic device may further comprise a UWB circuit, wherein the processor may be further configured, when executing the instructions, to identify a distance between the electronic device and the external electronic device through the UWB circuit.

According to an embodiment, the processor may be further configured, when executing the instructions, to obtain operation record information of the first wearable device stored in the memory, based on the operation record information of the first wearable device, to identify the first mode in which the first wearable device will operate.

According to an embodiment, the processor may be configured, when executing the instructions, to identify that the first wearable device is worn by a user, based on identifying that the first wearable device is worn by the user, perform a user authentication procedure, and based on the user authentication procedure, to identify the user wearing the first wearable device as the first user.

According to an embodiment, the first connection may be established based on Bluetooth, and the second connection may be established based on a wireless local area network.

According to an embodiment, the first wearable device may further comprise a first support part, a second support part, a connection part rotatably connecting the second support part relative to the first support part through the first actuator, wherein the first actuator may be attached to the first support part, wherein the second support part may be rotated relative to the first support part through torque provided from the first actuator.

A method of an electronic device according to various embodiments may comprise establishing a first connection with a first wearable device which comprises a first actuator and is worn by a first user of the electronic device, establishing a second connection with an external electronic device connected with a second wearable device which comprises a second actuator and is worn by a second user, while the first connection and the second connection are maintained, obtaining first movement information of the electronic device and second movement information of the external electronic device, based on the first movement information and the second movement information, identifying a first mode in which the first wearable device operates using the first actuator and a second mode in which the second wearable device operates using the second actuator, transmitting, through the first connection, a request signal for operating the first mode to the first wearable device, and transmitting, through the second connection, a request signal for operating the second mode to the external electronic device.

A non-transitory computer readable storage medium according to various embodiments may store one or more programs comprising instructions which cause the electronic device, when being executed by at least one processor of an electronic device with global positioning system (GPS) circuit and at least one communication circuit, to establish a first connection with a first wearable device which comprises a first actuator and is worn by a first user of the electronic device, to establish a second connection with an external electronic device connected with a second wearable device which comprises a second actuator and is worn by a second user, while the first connection and the second connection are maintained, to obtain first movement information of the electronic device and second movement information of the external electronic device, based on the first movement information and the second movement information, to identify a first mode in which the first wearable device operates using the first actuator and a second mode in which the second wearable device operates using the second actuator, to transmit, through the first connection, a request signal for operating the first mode to the first wearable device, and to transmit, through the second connection, a request signal for operating the second mode to the external electronic device.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
at least one memory configured to store instructions;
global positioning system (GPS) circuit;
at least one communication circuit; and
at least one processor operably coupled to the at least one memory, the GPS circuit, and the at least one communication circuit, wherein the at least one processor is configured, when executing the instructions, to:
establish a first connection with a first wearable device which comprises a first actuator and is worn by a first user of the electronic device,
establish a second connection with an external electronic device connected with a second wearable device which comprises a second actuator and is worn by a second user,
while the first connection and the second connection are maintained, obtain first movement information of the electronic device and second movement information of the external electronic device,
based on the first movement information and the second movement information, identify a first mode in which the first wearable device operates using the first actuator and a second mode in which the second wearable device operates using the second actuator,
transmit, through the first connection, a request signal for operating the first mode to the first wearable device, and
transmit, through the second connection, a request signal for operating the second mode to the external electronic device.

2. The electronic device of claim 1, wherein the first mode is set one of a plurality of modes for the first wearable device to output assist torque acting in a direction to assist movement of the first user using the first actuator,
wherein the second mode is set one of a plurality of modes for the second wearable device to output resist torque acting in a direction to resist movement of the second user using the second actuator,
wherein size of the torque output using the first actuator in the first mode is changed based on a posture of the first user, and
wherein size of the torque output using the second actuator in the second mode is changed based on a posture of the second user.

3. The electronic device of claim 2, wherein the first movement information is obtained from data related to locations of electronic device obtained by the GPS circuit,
wherein the second movement information is obtained from the external electronic device, and
wherein the processor is configured, when executing the instructions, to:
based on the first movement information and the second movement information, identify a distance between the electronic device and the external electronic device,
identify that the distance between the electronic device and the external electronic device is greater than or equal to a designated distance, and
based on identifying that the distance between the electronic device and the external electronic device is greater than or equal to the designated distance, identify the first mode and the second mode.

4. The electronic device of claim 3, wherein the processor is configured, when executing the instructions, to identify, based on the first movement information and the second movement information, that the external electronic device moving in a first direction is followed by the electronic device moving in the first direction.

5. The electronic device of claim 4, wherein the processor is configured, when executing the instructions, to identify, based on identifying that the external electronic device moving in the first direction is followed by the electronic device moving in the first direction, identify the first mode for resisting movement in the first direction and the second mode for assisting movement in the first direction.

6. The electronic device of any one of claims 1 to 5, wherein the processor is configured, when executing the instructions, to:
receive biological data of the first user from a third wearable device connected with the electronic device,
receive biological data of the second user from the external electronic device, and
identify, based on the biological data of the first user and the biological data of the second user, the first mode and the second mode.

7. The electronic device of any one of claims 1 to 6, wherein the processor is configured, when executing the instructions, to:
obtain, based on a user input of the first user, information on target speed,
identify movement speed of the electronic device identified based on the first movement information is less than the target speed, and
based on identifying that movement speed of the electronic device obtained based on the first movement information is less than the target speed, identify the first mode to increase the movement speed of the electronic device to the target speed.

8. The electronic device of any one of claims 1 to 7, wherein the processor is further configured, when executing the instructions, to:
based on the first movement information and a mode of the first wearable device set before transmitting the request signal for operating the first mode, identify torque caused by walking of the first user, and
based on the torque caused by the walking of the first user, identify the first mode.

9. The electronic device of any one of claims 1 to 8, wherein the processor is further configured, when executing the instructions, to establish connections with a plurality of external electronic devices operating as slave devices, located within a designated distance from the electronic device while the electronic device is operated as a master device,
wherein each of the plurality of external electronic devices are connected with each of a plurality of wearable devices.

10. The electronic device of claim 9, wherein the processor is configured, when executing the instructions, to:
receive, from each of the plurality of external electronic devices, movement information of the plurality of external electronic devices,
identify each mode in which the plurality of wearable devices will operate so that the plurality of external electronic devices move based on a designated interval, and
transmit a plurality of signals for the plurality of wearable devices to operate in each of the identified mode to each of the plurality of external electronic devices.

11. The electronic device of any one of claims 9 to 10, wherein the processor is configured, when executing the instructions, to:
receive information on a user of each of the plurality of external electronic devices from the plurality of external electronic devices,
obtain information on a target speed based on the information on the user of each of the plurality of external electronic devices and information on the first user,
based on the information on the target speed, identify each mode in which the plurality of external electronic devices to be operated,
transmit the plurality of signals for the plurality of wearable devices to operate in each of the identified mode to each of the plurality of external electronic devices.

12. The electronic device of claim 11, wherein the processor is configured, when executing the instructions, to:
obtain a user input for changing the target speed from the first user, and
based on the user input, change each mode in which the plurality of wearable devices will operate.

13. The electronic device of any one of claims 9 to 12, wherein the electronic device further comprises a rechargeable battery, and
wherein the processor is configured, when executing the instructions, to:
while the electronic device operates as a master device, receive information on remaining battery capacity from each of the plurality of external electronic devices,
while the electronic device operates as a master device, identify that remaining battery capacity of the battery comprised in the electronic device is less than or equal to a designated value,
based on identifying that the remaining battery capacity of the battery comprised in the electronic device is less than or equal to the designated value, identify an external electronic device with the largest remaining battery capacity among the plurality of external electronic devices, and
in response to identifying the external electronic device with the largest remaining battery capacity, perform signal exchange with the external electronic device with the largest remaining battery capacity so that the electronic device operates as a slave device and the external electronic device with the largest remaining battery capacity operates as a master device.

14. A method of an electronic device comprising:
establishing a first connection with a first wearable device which comprises a first actuator and is worn by a first user of the electronic device,
establishing a second connection with an external electronic device connected with a second wearable device which comprises a second actuator and is worn by a second user,
while the first connection and the second connection are maintained, obtaining first movement information of the electronic device and second movement information of the external electronic device,
based on the first movement information and the second movement information, identifying a first mode in which the first wearable device operates using the first actuator and a second mode in which the second wearable device operates using the second actuator,
transmitting, through the first connection, a request signal for operating the first mode to the first wearable device, and
transmitting, through the second connection, a request signal for operating the second mode to the external electronic device.

15. A non-transitory computer readable storage medium storing one or more programs, wherein the one or more programs comprising instructions, which, when being executed by at least one processor of an electronic device with global positioning system (GPS) circuit and at least one communication circuit, cause the electronic device to:
establish a first connection with a first wearable device which comprises a first actuator and is worn by a first user of the electronic device,
establish a second connection with an external electronic device connected with a second wearable device which comprises a second actuator and is worn by a second user,
while the first connection and the second connection are maintained, obtain first movement information of the electronic device and second movement information of the external electronic device,
based on the first movement information and the second movement information, identify a first mode in which the first wearable device operates using the first actuator and a second mode in which the second wearable device operates using the second actuator,
transmit, through the first connection, a request signal for operating the first mode to the first wearable device, and
transmit, through the second connection, a request signal for operating the second mode to the external electronic device.
